# EUROPÄISCHE PATENTSCHRIFT

(11) **EP 2 888 589 B1**
(45) Veröffentlichungstag und Bekanntmachung des Hinweises auf die Patenterteilung: **20.02.2019**
(21) Anmeldenummer: 13752592.9
(22) Anmeldetag: 22.08.2013
(51) Int. Cl.: G01N 33/50

(54) **VERFAHREN UND VORRICHTUNG ZUR UNTERSTÜTZUNG DER ERHALTUNG DER INSEKTENPOPULATION**
METHOD AND DEVICE FOR THE ASSISTANCE OF THE PRESERVATION OF THE POPULATION OF INSECTS
METHODE ET DISPOSITIF POUR SOUTENIR LE MAINTIEN DE LA POPULATION D'INSECTES

(30) Priorität: 24.08.2012 DE 102012016885; 24.10.2012 DE 102012020912
(43) Veröffentlichungstag der Anmeldung: 01.07.2015
(73) Patentinhaber: Reckhaus AG, 9056 Gais (CH)
(72) Erfinder: RECKHAUS, Hans-Dietrich, 9053 Teufen (CH)
(74) Vertreter: Tostmann, Holger Carl
(86) Internationale Anmeldenummer: PCT/EP2013/002527
(87) Internationale Veröffentlichungsnummer: WO 2014/029503

(56) Entgegenhaltungen:
- STOFFEL EFROM CAIO FABIO ET AL: "Side-Effects of Pesticides Used in the Organic System of Production on Apis mellifera Linnaeus, 1758", BRAZILIAN ARCHIVES OF BIOLOGY AND TECHNOLOGY, Bd. 55, Nr. 1, Januar 2012 (2012-01), Seiten 47-53, XP002716034,
- Petzoldt, C., Kovach, J, Engel, J: "Evaluating Pesticides for Their Impact on Beneficial Organisms", , 13. Juni 2010 (2010-06-13), XP002716035, The Ohio State University Gefunden im Internet: URL:http://web.archive.org/web/20100613122 321/http://nysipm.cornell.edu/publications /eiq/files/ben_org_eval_sum.pdf [gefunden am 2013-11-06]
- S. A. HASSAN ET AL: "Standard methods to test the side-effects of pesticides on natural enemies of insects and mites developed by the IOBC/WPRS Working Group 'Pesticides and Beneficial Organisms'", EPPO BULLETIN, Bd. 15, Nr. 2, 1. Juni 1985 (1985-06-01), Seiten 214-255, XP055009062, ISSN: 0250-8052, DOI: 10.1111/j.1365-2338.1985.tb00224.x
- "Verkehrsopfer, an die niemand denkt Insect Respect erforscht Insektentötung durch fahrende Autos", , 27. September 2013 (2013-09-27), XP055087115, Gefunden im Internet: URL:http://www.insect-respect.org/fileadmi n/downloads/Medien/mm_insectrespect_test20 13_20130927.pdf [gefunden am 2013-11-07]

## Beschreibung

Die vorliegende Erfindung betrifft ein Verfahren zur Unterstützung der Erhaltung der Insektenpopulation.

In vielen privaten und gewerblichen Bereichen werden Produkte zur Insektenbekämpfung verwendet. Durch diese werden Insekten und andere Arthropoden getötet und fehlen anschließend im Ökosystem.

Auch durch andere Produkte werden Insekten und andere Arthropoden getötet und damit dem Ökosystem entzogen. Dabei handelt es sich beispielsweise um Produkte, bei deren Anwendung ungewollt Insekten zu Schaden kommen, insbesondere infolge einer Kollision mit bewegten Produkten, insbesondere solchen, die vorwiegend zum Transport von Personen oder Gütern dienen. Als weiteres Beispiel für Produkte, bei deren Anwendung ungewollt Insekten zu Schaden kommen, sind auch Scheinwerfer zu nennen, welche durch ihr Licht Insekten anlocken, welche bei zu nahem Kontakt daran "verglühen".

In der Folge dieser gewollten oder ungewollten Produktwirkungen fehlen Insekten, welche insbesondere die Blüten bestäuben und als Nahrungsgrundlage für Vögel, Fische, Kriechtiere und Säugetiere dienen.

Der Aufsatz "Side-Effects of Pesticides Unsed in the Organic System of Production on Apis mellifera Linnaeus, 1758" von Stoffel Efrom et.al, erschienen im internationalen Journal Brazilian Archives of Biology and Technology (Vol. 55, n. 1, Seiten 47 - 53, Januar-Februar 2012, ISSN 1516-8913) betrifft die Evaluierung der Effekte von Pestiziden auf Honigbienen unter Laborbedingungen.

Der Posterbeitrag *"*Evaluating Pesticides for Thier Impact on Beneficial Organisms" von Petzoldt et al., abrufbar unter http://web.archive.org/web/20100613122321/http://nysipm.cornell.edu/publications/eiq/file s/ben_org_eval_sum.pdf betrifft ein multikriterielles Ranking-System zur Einordnung von Pestiziden hinsichtlich er Effekte unterschiedlicher Pestizide auf verschiedene Insektenarten bzw. Mikroorganismen.

Der Aufsatz *"*Standard methods to test the side-effects of pesticides on natural enemies of insects and mites developed by the IOBC/WPRS Working Group 'Pesticides and Beneficial Organisms"'von Hassan et al. erschienen in EPPO Bulletin 15, S. 214 - 255, ISSN 0250-8052, 1980 stellt insgesamt neunzehn Laborversuchsverfahren, zwölf Teil-Feldversuchsverfahren und fünf Feldversuchsverfahren vor, welche eingesetzt werden können, um die Auswirkungen von Pestiziden auf nützliche Organismen zu untersuchen.

Der Erfindung liegt daher die Aufgabe zugrunde, den Verlust von Insekten zu kompensieren.

Dies wird erfindungsgemäß durch die Lehre der unabhängigen Ansprüche erreicht. Zu bevorzugende Weiterbildungen der Erfindung sind Gegenstand der Unteransprüche.

Bei dem erfindungsgemäßen Verfahren zur Unterstützung der Erhaltung der Insektenpopulation wird zunächst die letale Wirkung einer Produkteinheit auf eine erste Insektenpopulation ermittelt und die Insektenpopulationsdifferenz aufgrund der Wirkung einer vorbestimmten Menge von Produkteinheiten auf die erste Insektenpopulation bestimmt.

Dann wird eine zweite Insektenpopulation, die Ist-Insektenpopulation auf einer vorbestimmten Fläche ermittelt und eine Soll-Insektenpopulation auf dieser vorbestimmten Fläche bestimmt, wobei die Soll-Insektenpopulation die Summe aus der Ist-Insektenpopulation auf der vorbestimmten Fläche und der wahlweise um einen Faktor vergrößerten oder verringerten Insektenpopulationsdifferenz abgeleitet ist.

Für die Soll-Insektenpopulation wird dann eine Lebensgrundlage auf der vorbestimmten Fläche durch Anpassen der biologischen Beschaffenheit der vorbestimmten Fläche geschaffen, dadurch gekennzeichnet, dass die Wertigkeit, also die biologische Beschaffenheit der vorbestimmte Fläche mithilfe einer Einbringung von Biostrukturen in die Fläche erhöht wird, wobei die in die Fläche eingebrachten Biostrukturen aus einer Gruppe ausgewählt sind, die Anhügelungen, Steinhaufen, Asthaufen, Trockenmauern, Steinkörbe, Kleingewässer, Bepflanzung wie insbesondere Bäume und Sträucher, Fassadenbegrünungen, Insektenhotels und Bachoffenlegung aufweist.

Produkte vielfältiger Art wirken auf eine Insektenpopulation tödlich, also letal. Beispielsweise werden mit Produkten zur Insektenbekämpfung Insekten und andere Arthropoden wie beispielsweise Spinnen aus einem Bereich, in welchem diese beispielsweise aus hygienischen Gründen unerwünscht sind, entfernt und dabei durch diese Produkte üblicherweise getötet. So weisen Produkte zur Insektenbekämpfung gewöhnlich eine gewisse letale Wirkung auf, d.h. ein gewisses Potenzial zum Abtöten einer bestimmten Menge von für dieses Produkt charakteristischen Insektenarten. Dieses Potenzial einer Produkteinheit eines Mittels zur Insektenbekämpfung entspricht dessen letaler Wirkung auf die Insektenpopulation. Vorzugsweise wird bei der Bestimmung der letalen Wirkung eines Produktes auch der Zeitraum berücksichtigt, in welchem das Produkt angewendet wird oder wirkt. Abhängig vom Produkt fließt bei der Bestimmung von dessen letaler Wirkung bevorzugt auch der Zeitpunkt (Jahreszeit, tagsüber, nachts, etc.) und/ oder die Umgebung (Gebäude, im Freien, etc.) ein, in welcher die Produkteinheit eingesetzt wird.

Auch bewegte Produkte, wie solche zum Transport von Personen oder Gütern wie insbesondere Kraftfahrzeuge, Züge, Binnenschiffe, Flugzeuge beim Start oder bei der Landung oder auch Gondeln von Bergbahnen und sonstigen Fahrgeschäften oder auch alternativ angetriebene Fahrzeuge wie Fahrräder wirken auf eine Insektenpopulation letal. Bewegen sich diese Produkte mit insbesondere höheren Geschwindigkeiten, so kollidieren häufig und in großer Menge Insekten und andere Arthropoden insbesondere mit den Frontpartien dieser Produkte und werden dabei zumeist getötet. Folglich weisen auch solche Produkte eine gewisse letale Wirkung, d.h. ein gewisses Potential zum Töten einer bestimmten Menge von Insekten auf. Auch bei der Bestimmung der letalen Wirkung solcher bewegter Produkte werden vorzugsweise neben der Wirkungsweise auch der Anwendungszeitpunkt, die Anwendungsdauer und der Anwendungsbereich betrachtet. Die Anteile der getöteten Insektenarten hängen von der Insektenpopulation in dem Gebiet ab, in welchem ein solches Produkt bewegt wird. Das Potenzial einer Produkteinheit zum Töten einer bestimmten Menge von Insekten entspricht deren letaler Wirkung auf die Insektenpopulation.

Auch viele weitere Produkte, welche sich insbesondere schnell bewegende Teile aufweisen, wie beispielsweise Windräder, mit deren Rotorblättern Insekten kollidieren, haben eine letale Wirkung auf die Insektenpopulation. Ebenso führen viele Produkte mit für vielfältigste Zwecke bewegten Teilen zu tödlichen Fallen für Insekten. So sind beispielsweise zur Grünfutterernte verwendete Kreiselmähwerke so leistungsfähig, dass aufgrund der hohen Mähgeschwindigkeiten auch größere Insekten wie Bienen oder Schmetterlinge die Grünfläche oft nicht mehr rechtzeitig verlassen können und im Grünfutter ersticken.

Eine Vielzahl weiterer Produkte weist neben der gewünschten Produktwirkung auch eine ungewünschte letale Wirkung auf die Insektenpopulation aus. So lockt beispielsweise das Licht von Scheinwerfern, insbesondere von Hochleistungsbeleuchtungseinrichtungen wie Flutlichtanlagen in Sportstadien oder an Flughäfen oder auch das Licht von Straßenbeleuchtungen viele Insekten an, welche bei zu nahem Kontakt mit diesen Beleuchtungsanlagen insbesondere durch deren Wärmeentwicklung umkommen.

Die hier vorausgehend beispielhaft genannten sowie eine Vielzahl weiterer Produkte, bei deren Anwendung Insekten oder andere Anthropoden zu Schaden kommen, weisen folglich ein gewisses Potenzial zum Töten einer bestimmten Menge von Insekten auf. Das Potenzial einer Produkteinheit entspricht deren letaler Wirkung auf die Insektenpopulation. Bei der Ermittlung der letalen Wirkung einer Produkteinheit werden zur Vereinfachung der Anwendung des Verfahrens vorzugsweise gemittelte Werte für das Produkt oder für ein ähnliches Produkt eingesetzt, wobei Unterschiede in der letalen Wirkung wie beispielsweise eine größere oder kleinere Frontfläche eines Fahrzeugs bevorzugt mittels Korrekturfaktoren berücksichtigt werden.

Aufgrund dieser letalen Wirkung einer vorbestimmten Menge von Produkteinheiten kann die durch die Anwendung bzw. durch den Einsatz dieser Menge von Produkteinheiten resultierende Insektenpopulationsdifferenz bestimmt werden.

So werden beispielsweise bei einer Produkteinheit einer Klebeinsektenfalle mit etwa 100 cm² Klebefläche durchschnittlich 150 Fliegen mit etwa 720 mg gefangen. 100.000 Dosen Insektenspray (400ml) töten durchschnittlich beispielsweise etwa 50 kg Insekten, so dass die Insektenpopulationsdifferenz von 100.000 Dosen eines Insektensprays etwa 50 kg Insekten beträgt.

Häufig entfalten insbesondere Produkte zur Insektenbekämpfung ihre letale Wirkung im Wesentlichen an Insektenarten, die für ihr Ökosystem wenig wichtig und damit wenig wertvoll sind, wie beispielsweise Stubenfliegen.

Bei einer Produkteinheit eines Personenkraftfahrzeugs mit etwa 2 m² Frontfläche werden gemittelt über die Jahreszeiten bei einer durchschnittlichen Jahreskilometerleistung von 18.000 km beispielsweise mehr als 30 g Insekten pro Jahr getötet. Die Insektenpopulationsdifferenz aufgrund der Wirkung von bewegten Produkten wird folglich insbesondere beeinflusst durch deren Anströmfläche, deren Bewegungsstrecke und Bewegungsgeschwindigkeit sowie durch die Dichte der Insektenpopulation in dem Gebiet, in welchem sich das Produkt bewegt.

Jede Fläche, insbesondere außerhalb von Gebäuden, ist natürlich von einer gewissen Menge üblicherweise verschiedener Arten von Insekten besiedelt und weist damit eine Ist-Insektenpopulation auf. Für die Ausführung des erfindungsgemäßen Verfahrens zur Unterstützung der Erhaltung der Insektenpopulation wird auf einer vorbestimmten Fläche die Ist-Insektenpopulation ermittelt. Insbesondere kann die Ist-Insektenpopulation auf einer Fläche aufgrund der biologischen Beschaffenheit, die auch als Wertigkeit bezeichnet wird, ermittelt werden. Dabei wird aufgrund von Erfahrungswerten für Flächen, die gleiche oder zumindest ähnliche biologische Beschaffenheit aufweisen, die Größe der Insektenpopulation aufgrund der Häufigkeit bestimmter unterschiedlicher Insektenarten bzw. anderer Anthropoden pro Quadratmeter und Jahr auf einer Fläche eingeschätzt. Auch hier ist eine Anpassung des ermittelten Werts mit Hilfe von Kennzahlen möglich.

Ausgehend von der Ist-Insektenpopulation auf einer Fläche ist in Verbindung mit der Insektenpopulationsdifferenz, die aus der Wirkung einer vorbestimmten Menge von Produkteinheiten resultiert, eine Soll-Insektenpopulation für diese vorbestimmte Fläche bestimmbar, welche eine Erhaltung der Insektenpopulation unterstützt. Dabei kann die Soll-Insektenpopulation die Summe aus der Ist-Insektenpopulation und der Insektenpopulationsdifferenz betragen. Ferner kann die Soll-Insektenpopulation auch durch eine Summe der Ist-Insektenpopulation und der um einen Faktor vergrößerten oder verringerten Insektenpopulationsdifferenz bestimmt werden. Üblicherweise wird bei einer solchen Berechnung als Einheit der Insektenpopulation das Körpergewicht der Insekten im Milligramm pro Quadratmeter Fläche verwendet.

In eine derartige Berechnung kann vorzugsweise auch ein Korrekturfaktor zur Berücksichtigung eines ökologischen Werts der Artenvielfalt einfließen. Bei Verwendung eines derartigen Korrekturfaktors könnte dann besonders wertvollen bzw. nützlichen Insektenarten ein höherer Wert des Korrekturfaktors und damit ein höherer Ausgleichswert zugewiesen werden als Insektenarten, die für die Artenvielfalt des Ökosystems weniger wertvoll eingeschätzt werden. So kann beispielsweise Bienen oder Ameisen ein für die Artenvielfalt höherer Korrekturfaktor zugewiesen werden als beispielsweise Stuben- oder Fruchtfliegen.

Damit die vorbestimmte Fläche die Lebensgrundlage für eine Soll-Insektenpopulation bildet, muss diese Fläche zumindest eine vorbestimmte biologische Beschaffenheit, also Wertigkeit, aufweisen. Daher wird in einem weiteren Schritt des erfindungsgemäßen Verfahrens die biologische Beschaffenheit der Fläche so angepasst, dass diese eine Lebensgrundlage für die ermittelte Soll-Insektenpopulation auf dieser vorbestimmten Fläche schafft. Dabei wird die Wertigkeit der biologischen Beschaffenheit so verändert, dass auf der Fläche eine Lebensgrundlage für eine größere Insektenpopulation, der Soll-Insektenpopulation geschaffen wird. Daraus resultiert dann eine Ansiedelung einer zusätzlichen Masse von Insekten, so dass die vorbestimmte Fläche die ermittelte Soll-Insektenpopulation aufweist. Ausgehend von der Insektenpopulationsdifferenz aufgrund der Wirkung einer vorbestimmten Menge von Produkteinheiten wird durch dieses Verfahren die Erhaltung der Insektenpopulation im Ökosystem unterstützt.

Die letale Wirkung einer Produkteinheit auf die Insektenpopulation ist dabei vorzugsweise mechanisch und/ oder chemisch.

Unter mechanische Produkteinheiten zur Insektenbekämpfung fallen insbesondere Fliegenklatschen oder Klebefallen, wobei Klebefallen neben der mechanischen Festhaltewirkung auch häufig chemische Wirkstoffe zur schnelleren Tötung oder zum Anlocken von Insekten aufweisen.

Unter mechanische Produkteinheiten fallen insbesondere bewegte Produkte wie ein- oder zweispurige Kraftfahrzeuge, Personen- oder Güterzüge, startende und landende Flugzeuge, Binnenschiffe, Gondeln in Bergbahnen und sonstigen Fahrgeschäften oder auch alternativ angetriebene Fahrzeuge wie Fahrräder.

Als rein chemisch werden Produkteinheiten verstanden, deren Wirkung auf einem in einem Raum oder auf eine Fläche ausgebrachten Wirkstoff beruht, wie beispielsweise Insektensprays. Vorzugsweise werden neben mechanischen und/ oder chemischen Wirkstoffen bei speziellen Anwendungen auch biologische Wirkstoffe, bevorzugt gemeinsam mit mechanischen und oder chemischen Wirkstoffen verwendet.

Insbesondere bestimmt sich die letale Wirkung einer bevorzugten Produkteinheit auf die Insektenpopulation aufgrund der Toxizität eines vorzugsweise chemischen Wirkstoffs und der auf eine Insektenpopulation wirkenden Wirkstoffmenge. Dabei ist die letale Wirkung insbesondere umso größer, je wirksamer der Wirkstoff, je höher die Wirkstoffkonzentration und je größer die aufgebrachte bzw. freigesetzte Wirkstoffmenge ist.

Ferner bestimmt sich die letale Wirkung einer bevorzugten chemisch und/ oder mechanisch wirkenden Produkteinheit auf die Insektenpopulation aufgrund der Oberflächeneigenschaften und/ oder der Geometrie der Produkteinheit. Bei Produkteinheiten mit diesen Eigenschaften handelt es sich insbesondere um Insektenfallen, in welche Insekten von der Farbe oder dem Geruch der Produkteinheit angelockt werden und dort beispielsweise in eine unentrinnbare Falle geraten, wie ein Gefäß mit einer flüssigen Lösung, deren Oberflächenspannung herabgesetzt ist, eine Klebefläche oder einem für ein Insekt nicht verlassbaren Hohlraum.

Auch die Oberflächeneigenschaften und/ oder die Geometrie stellen bevorzugte Einflussfaktoren für die letale Wirkung einer vorzugsweise mechanisch wirkenden, bewegten Produkteinheit auf die Insektenpopulation dar. So kann insbesondere die Farbe der Oberfläche einer bewegten Produkteinheit Insekten veranlassen, sich auf diese zu und damit in die Bewegungsbahn der Produkteinheit zu bewegen, in welcher sie anschließend mit dem bewegten Produkt kollidieren. Ferner ist die Geometrie, z. B. in der Ausprägung einer geschlossenen Fläche oder einer durchbrochenen Fläche wie z. B. ein feinmaschiges Netz und die Größe, insbesondere der Oberfläche der Stirnfläche des bewegten Produkts insbesondere aufgrund der unterschiedlichen Luftanströmeigenschaften ein wichtiger Einflussfaktor für die Menge der mit dem Produkt kollidierenden Insekten.

Des Weiteren handelt es sich bei einer bevorzugten Ausführungsform des Verfahrens um Produkteinheiten, deren letale Wirkung auf die Insektenpopulation aufgrund einer negativen Auswirkung auf die Fortpflanzungsfähigkeit der Insekten bestimmt wird. Durch eine solche Produkteinheit wird eine Insektenpopulationsdifferenz auf die Weise geschaffen, dass durch die zerstörte Fortpflanzungsfähigkeit der Insekten die Insektenpopulationsdifferenz erst zeitlich versetzt zur Anwendung der Produkteinheit entsteht.

Bei jeder der vorgenannten Art von Produkteinheiten kann die letale Wirkung auf die Insektenpopulation durch wenigstens ein Lockmittel verstärkt sein. Die Wirkung verstärkt ein Lockmittel dadurch, dass die Insekten von der Produkteinheit angelockt werden, so dass die Produkteinheit auf eine größere Insektenpopulation wirken kann, als in dem Bereich angesiedelt ist, in welchem die Produkteinheit angewendet wird bzw. seine Wirkung entfaltet.

Ein solches Lockmittel kann insbesondere im Falle einer chemisch wirkenden Produkteinheit ein Duftstoff sein, welcher auf eine bestimmte oder mehrere Arten von Insekten wirkt. Insbesondere bei bewegten Produkteinheiten kann die Farbe wie insbesondere ein heller Gelbton oder eine Beleuchtung eine Lockwirkung auf Insekten ausüben. Dies trifft insbesondere auf Scheinwerfer bewegter Produkteinheiten wie Fahrzeuge oder allgemein auf alle Arten von Beleuchtungseinrichtungen zu.

Bei bewegten Produkteinheiten bestimmt sich die letale Wirkung insbesondere aus der Größe einer im Wesentlichen senkrecht zur Bewegungsrichtung stehenden Anströmfläche und der Größe einer durch diese Produkteinheit mit einer insbesondere über einem Geschwindigkeits-Grenzwert liegenden Geschwindigkeit zurückgelegten Strecke. Der Geschwindigkeits-Grenzwert einer bewegten Produkteinheit ist insbesondere auch von deren Geometrie abhängig ist. Bei einem Kraftfahrzeug liegt dieser bei etwa 50 km/h.

Die letale Wirkung einer solchen Produkteinheit steigt mit größeren Werten insbesondere von Anströmfläche, zurückgelegter Strecke und Geschwindigkeit. Ab dem Geschwindigkeitsgrenzwert werden sich in der Luft befindende Insekten im Wesentlichen nicht mehr von einem Luftstrom um eine Anströmfläche herumgelenkt oder prallen dort für diese unschädlich sanft auf, sondern kollidieren so mit der Anströmfläche, dass diese beim Aufprall getötet werden.

Die Ist-Insektenpopulation auf einer vorbestimmten Fläche wird bei einer bevorzugten Ausführungsform des Verfahrens aufgrund der Wertigkeit der vorbestimmten Fläche bestimmt. Die nachfolgende Tabelle zeigt eine bevorzugte Klassifikation von Flächen außerhalb von Gebäuden, für welche verschiedene Wertstufen vergeben werden. Bei den angegebenen Werten handelt es sich um das Gewicht einer auf einem Quadratmeter Fläche in der Vegetation lebenden Insektenpopulation innerhalb eines Jahres.

| **Wertstufe 0** | **Wertstufe I** | **Wertstufe II** | **Wertstufe III** | **Wertstufe IV** | **Wertstufe V** |
|---|---|---|---|---|---|
| *keine Bedeutung* | *minimale Bedeutung* | *geringe Bedeutung* | *durchschnittliche Bedeutung* | *hohe Bedeutung* | *sehr hohe Bedeutung* |
| < 800 mg/m² | 800-2.000 mg/m² | 2.000 - 4.000 mg/m² | 4.000 - 8.000 mg/m² | 8.000 - 12.000 mg/m² | > 12.000 mg/m² |
| asphaltierte Fläche | Komposthaufen | Insektenhotel | Einzelbaum | Trockenmauer, Steinkörbe | Kleingewässer |
| Ziegeldach | | Nistkästen | Fassadenbegrünung | Steinhaufen, Asthaufen | Bachoffenlegung |
| Flachdach mit Bitumenabdichtung | | | ökologischer Parkplatz | Hecke, Gehölze | |
| Pflasterfläche | | | | Ruderalfläche | |
| | | | | extensives Flachdach | |
| | | | | Obstgarten | |

Insbesondere ist mit steigender Wertstufe von einer höherer Artenvielfalt und damit von einem höheren Gewichtsanteil an Insektenarten auszugehen, welche einen hohen Wert für ihr Ökosystem aufweisen.

Soll durch das erfindungsgemäße Verfahren die Erhaltung von Insektenpopulationen im Boden unterstützt werden, so sind die hiervon abweichenden Gewichte von im Boden lebenden Insekten und gegebenenfalls einer bestimmten Insekten- oder Arthropodenart, und insbesondere deren Wert im Ökosystem, heranzuziehen.

Vorzugsweise wird mithilfe solcher Wertstufen die Wertigkeit einer Fläche einfach und schnell ermittelt. Zum Ermitteln der Wertigkeit einer bestehenden Fläche wird diese bevorzugt in eine Wertstufe klassifiziert. Andererseits können ausgehend von den Eigenschaften einer Fläche mit vorbekannter Wertstufe die Maßnahmen zum Anpassen der Fläche ermittelt werden, die erforderlich sind, damit diese zu einer Fläche mit gewünschter Wertstufe aufgewertet werden kann. Dabei ist jedoch zu berücksichtigen, dass die tatsächliche Insektenpopulation einer Fläche insbesondere abhängig von der klimatischen Lage, der Bodenbeschaffenheit und der Nutzung der Fläche zum Teil auch deutlich von in allgemeinen Tabellen angegebenen Werten abweicht, so dass eine hierfür bevorzugt eine passende Tabelle gewählt wird.

Bei einer bevorzugten Ausführungsform des Verfahrens wird die Ist-Insektenpopulation auf einer vorbestimmten Fläche mithilfe wenigstens eines Sensors erfasst. Dabei kann ein Sensor vorgesehen sein, welcher die Insekten unmittelbar selbst erfasst. Dies bietet sich insbesondere dann an, wenn die Ist-Insektenpopulation einer bestimmten Insektenart erfasst werden soll. Es ist jedoch - abhängig von biologischen Zusammenhängen - auch möglich, mittels der Erfassung der Ist-Insektenpopulation einer bestimmten Insektenart die Ist-Insektenpopulation einer weiteren, insbesondere verwandten und/ oder den gleichen Lebensraum bevorzugenden Insektenart oder auf die gesamte Ist-Insektenpopulation der Fläche zu bestimmen.

Ein bevorzugt geeigneter Sensor erfasst beispielsweise die Menge der sich im Messbereich bewegenden Ameisen oder ermittelt die Ameisenpopulation auf einer Fläche aufgrund der Zahl und Größe von erfassten Ameisenhaufen.

Bei einer weiter bevorzugten Ausführungsform des Verfahrens werden vorzugsweise mittels Sensoren Parameter erfasst, aufgrund deren Werten die Wertigkeit der vorbestimmten Fläche ermittelt und die Ist-Insektenpopulation auf dieser Fläche bestimmbar ist. Ein geeigneter Sensor weist vorzugsweise bildgebende Erfassungsmittel auf. Als Parameter, aufgrund welchen diese Wertigkeit bestimmbar ist, eignen sich insbesondere der Bewuchs, die Feuchtigkeit des Bodens und/ oder des Bewuchses, der Sauerstoffgehalt und/ oder die Schadstoffbelastung der Luft, die durchschnittliche Temperatur, die durchschnittliche Sonneneinstrahlung, die durchschnittliche Windgeschwindigkeit, die farblichen Eigenschaften des Bewuchses, die Bodenbeschaffenheit oder das Vorhandensein von Biostrukturen wie Gewässer oder Steinhaufen. Auch die Menge und/oder die Verhältnisse im Vorkommen verschiedener Insektenarten mit unterschiedlichem ökologischen Wert können als Parameter in die Bestimmung der Wertigkeit der vorbestimmten Fläche herangezogen werden. Anhand eines oder mehrerer dieser Parameter kann einerseits eine Klassifizierung der Fläche und andererseits eine mögliche Veränderung der Ist-Insektenpopulation im zeitlichen Verlauf erfasst werden.

Ausgehend von der Ist-Insektenpopulation auf der vorbestimmten Fläche wird mit der Insektenpopulationsdifferenz, die aus der Wirkung einer vorbestimmten Menge von Produkteinheiten resultiert, mittels geeigneter Bestimmungsverfahren, welche bevorzugt auch Korrekturfaktoren berücksichtigen, die Soll-Insektenpopulation dieser Fläche ermittelt.

Bei einer bevorzugten Ausführungsform des Verfahrens wird ausgehend von der Soll-Insektenpopulation die erforderliche Wertigkeit der vorbestimmten Fläche ermittelt. Auch hierzu wird bevorzugt ein Klassifikationssystem verwendet, welches auch bei der Bestimmung der Ist-Insektenpopulation aufgrund der Wertigkeit der bestehenden Fläche bestimmt wurde, und welches bevorzugt die Insektenpopulationen auf einer Fläche einer klassifizierten Wertstufe beinhaltet. Mithilfe eines solchen Klassifikationssystems kann die erforderliche Wertigkeit der vorbestimmten Fläche zum Schaffen einer Lebensgrundlage für die Soll-Insektenpopulation einfach und schnell bestimmt werden.

Das Potential einer Fläche wird bei einer bevorzugten Ausführungsform des Verfahrens aus der Differenz des Produkts aus der Wertigkeit der Fläche und der darauf lebenden Insektenpopulation im Endzustand und dem Produkt aus der Wertigkeit der Fläche und der darauf lebenden Insektenpopulation im Ursprungszustand ermittelt. Das Potential wird dabei vorzugsweise mittels Korrekturfaktoren entsprechend an weitere Einflussgrößen angepasst. So kann ein Faktor ermittelt werden, welcher direkt proportional zur Größe der Fläche ist. Ein solcher Faktor ergibt sich vorzugsweise aus dem Quotienten der Größe der Insektenpopulationsdifferenz und dem Potential der Fläche.

Bei einer anderen bevorzugten Ausführungsform des Verfahrens werden die erforderlichen Ausgleichmaßnahmen ermittelt. Dies erfolgt dadurch, dass die Ist-Insektenpopulation durch das Produkt aus Soll-Insektenpopulation und Insektenpopulationsdifferenz geteilt wird. Der berechnete Faktor stellt einen Wert für die erforderliche Anpassung der biologischen Beschaffenheit der Fläche dar.

Bei einer weiter bevorzugten Ausführungsform des Verfahrens ist zur Bestimmung einer Soll-Insektenpopulation ausgehend von der Ist-Insektenpopulation auf der vorbestimmten Fläche und der ermittelten Insektenpopulationsdifferenz eine Korrektur mit einem oder mehreren Faktoren vorgesehen. Dabei werden vorzugsweise Faktoren verwendet, durch welche insbesondere der zeitliche Versatz zwischen der Anwendung der Produkteinheit und der Anpassung der biologischen Beschaffenheit der vorbestimmten Fläche berücksichtigt wird. Ein weiterer bevorzugter Faktor berücksichtigt die Wirkungsentfaltung der Anpassung, d. h. eine zusätzliche Korrektur der Zeit bis wann der Ausgleichslebensraum etwa seine vorgesehene ökologische Wirkung (Biodiversität, Stabilisierung der Vegetation) entfaltet hat. Ein weiterer Korrekturfaktor berücksichtigt vorzugsweise die positive Wirkung der Strukturvielfalt der biologischen Beschaffenheit der vorbestimmten Fläche nach der Anpassung.

Die Strukturvielfalt wird insbesondere durch das Einbringen von Biostrukturen in eine Fläche wie Anhügelungen, Steinhaufen, Asthaufen, Trockenmauern, Steinkörbe, Kleingewässer, Bepflanzung wie insbesondere Bäume und Sträucher, Fassadenbegrünungen, Insektenhotels und Bachoffenlegung erhöht. Dadurch wird insbesondere die zeitliche Wirkungsentfaltung der Fläche als Lebensgrundlage einer Insektenpopulation verbessert und die Artenvielfalt und Quantität an Insekten auf der vorbestimmten Fläche des Lebensraums erhöht. Gleichzeitig wird die Wertigkeit der vorbestimmte Fläche mithilfe der Einbringung von Biostrukturen erhöht, so dass die Fläche als Lebensgrundlage für eine größere Insektenpopulation geeignet ist.

Insbesondere wenn es sich bei der betrachteten Insektenpopulation um Insekten handelt, deren Lebensgrundlage im Zusammenhang mit der Bodenbeschaffenheit steht, kann die Wertigkeit der vorbestimmten Fläche mithilfe einer Veränderung der Bodenbeschaffenheit erhöht werden und durch eine derartige Anpassung der biologischen Beschaffenheit der Fläche die Ansiedelung einer Soll-Insektenpopulation bewirkt werden.

Ferner wird auch eine Vorrichtung zum Einsatz bei der Ausführung des Verfahrens zur Unterstützung der Erhaltung der Insektenpopulation vorgeschlagen. Diese weist eine Wirkungserfassungseinrichtung zum Erfassen der letalen Wirkung einer Produkteinheit auf die Insektenpopulation auf, welche vorzugsweise aus wenigstens einem erfassten Wert die letale Wirkung einer Produkteinheit ableitet. Ferner weist die Vorrichtung eine Mengenerfassungseinrichtung auf, zum Erfassen der Menge der betrachteten Produkteinheiten sowie eine Bestimmungseinrichtung zum Bestimmen der Insektenpopulationsdifferenz infolge der Anwendung der Menge der Produkteinheiten.

Die erfindungsgemäße Vorrichtung weist weiter wenigstens eine Ist-Erfassungseinrichtung zum Erfassen der Ist-Insektenpopulation auf einer vorbestimmten Fläche auf. In eine Recheneinrichtung der Vorrichtung sind Werte aus den weiteren Einrichtungen der Vorrichtung einlesbar. Die Recheneinrichtung leitet wenigstens aus den Werten für die Insektenpopulationsdifferenz und für die Ist-Insektenpopulation auf der vorbestimmten Fläche die Soll-Insektenpopulation auf der vorbestimmten Fläche ab.

Die Wirkungserfassungseinrichtung der Vorrichtung zum Einsatz bei der Ausführung des Verfahrens dient zum Erfassen der letalen Wirkung einer Produkteinheit auf die Insektenpopulation. Dabei wird die letale Wirkung einer Produkteinheit vorzugsweise auf das Lebendgewicht der mit einer Produkteinheit durchschnittlich getöteten Insekten bezogen. Bei der Vorrichtung zum Erfassen der letalen Wirkung kann es sich je nach Ausführungsform der Vorrichtung um ein Eingabegerät wie eine Tastatur oder einen Touch Screen handeln, mit dem ein vorhandener Wert, welcher die letale Wirkung einer Produkteinheit repräsentiert, einlesbar ist. Bei der Vorrichtung kann sich aber auch um eine Messvorrichtung handeln, mit welcher die Wirkstoffe und vorzugsweise die Wirkstoffmenge eines Produkts erfasst wird und in Verbindung mit bevorzugt in der Wirkungserfassungseinrichtung hinterlegten Daten und vorzugsweise weiterer Angaben insbesondere betreffend die Wirkungsweise und/ oder die Anwendung des Produkts, die letale Wirkung einer Produkteinheit abgeleitet wird. Ein in der Wirkungserfassungseinrichtung bestimmter Wert für die letale Wirkung einer Produkteinheit wird dort vorzugsweise für die Recheneinrichtung einlesbar bereitgestellt.

Die Vorrichtung weist weiter eine Mengenerfassungseinrichtung zum Erfassen der Menge der betrachteten Produkteinheiten auf. Dabei kann es sich je nach Ausführungsform der Vorrichtung insbesondere um ein Eingabegerät wie eine Tastatur oder einen Touch Screen handeln, mittels welchem ein Wert für die Menge der Produkteinheiten in die Vorrichtung eingebbar bzw. einlesbar ist.

Die Vorrichtung weist weiter eine Bestimmungseinrichtung auf, die zum Bestimmen der Insektenpopulationsdifferenz infolge der Anwendung der Menge der Produkteinheiten dient. Die Bestimmung der Insektenpopulationsdifferenz erfolgt dabei unter Verwendung des Werts für die letale Wirkung der Produkteinheit. Vorzugsweise werden bei dieser Bestimmung auch in der Bestimmungseinrichtung hinterlegte Daten für die mit einer Produkteinheit getöteten Insekten verwendet, wie insbesondere das Gewicht oder die Populationsdichte eines Insekts oder dessen Fortpflanzungscharakteristik.

Die erfindungsgemäße Vorrichtung weist weiter wenigstens eine Ist-Erfassungseinrichtung auf, zum Erfassen der Ist-Insektenpopulation auf einer vorbestimmten Fläche. Neben Eingabegeräten wie einer Tastatur oder einem Touch Screen, mittels welchem ein Wert insbesondere für die Wertigkeit der vorbestimmten Fläche einlesbar bzw. eingebbar ist, aus welchem insbesondere mit Hilfe hinterlegter Daten die Ist-Insektenpopulation bestimmt wird, kann die Vorrichtung vorzugsweise auch unmittelbare Ist-Erfassungseinrichtungen wie Sensoren aufweisen.

Wie bereits im Zusammenhang mit dem Verfahren zur Unterstützung der Erhaltung der Insektenpopulation beschrieben wurde, ist dabei vorzugsweise ein Sensor vorgesehen, welcher die Insekten unmittelbar selbst erfasst. Eine bevorzugte Ist-Erfassungseinrichtung ist so ausgestaltet, dass damit aufgrund der erfassten Daten hinsichtlich der Ist-Insektenpopulation einer bestimmten Insektenart die Ist-Insektenpopulation einer weiteren Insektenart oder die gesamte Ist-Insektenpopulation der Fläche bestimmbar ist.

Bei einer weiter bevorzugten Ausführungsform der Vorrichtung weist die Ist-Erfassungseinrichtung einen oder mehrere Sensoren auf, welche Parameter erfassen, welche die Wertigkeit der vorbestimmten Fläche ermitteln. Insbesondere in Verbindung mit vorzugsweise in der Vorrichtung hinterlegten Daten bestimmt die Ist-Erfassungseinrichtung bevorzugt die Ist-Insektenpopulation auf der vorbestimmten Fläche. Als Sensoren einer solchen Ist-Erfassungseinrichtung eignen sich insbesondere Feuchtigkeitssensoren, Gas- und Feststoffsensoren, Temperatursensoren, Helligkeits- bzw. Strahlungserfassungssensoren, Bewegungssensoren insbesondere zum Erfassen einer Windgeschwindigkeit, Farbsensoren oder Kameras vorzugsweise mit Bilderkennung.

In eine Recheneinrichtung der Vorrichtung sind Werte aus den weiteren Einrichtungen der Vorrichtung einlesbar. Mehrere Elemente der Vorrichtung können dabei in einer oder mehreren Funktionseinheiten der Vorrichtung untergebracht sein. Vorzugsweise sind in der Rechnereinrichtung auch Funktionseinheiten der Wirkungserfassungseinrichtung, der Mengenerfassungseinrichtung, der Bestimmungseinrichtung oder der Ist-Erfassungseinrichtung angeordnet. Diese Funktionseinheiten können jedoch auch örtlich unabhängig voneinander angeordnet sein und sind vorzugsweise zumindest zeitweilig über Funk- oder Drahtnetze miteinander verbunden.

Die Recheneinrichtung bestimmt wenigstens aus den Werten für die Insektenpopulationsdifferenz und für die Ist-Insektenpopulation die Soll-Insektenpopulation auf der vorbestimmten Fläche. Hierfür greift diese bevorzugt zumindest teilweise auf von anderen Einrichtungen der Vorrichtung ermittelte Werte sowie insbesondere auf in der Rechnereinrichtung hinterlegte Daten sowie Rechenalgorithmen zurück.

In der Recheneinrichtung der Vorrichtung sind Maßnahmen zur Anpassung der biologischen Bodenbeschaffenheit hinterlegt. Ausgehend von der erfassten Ist-Insektenpopulation auf einer vorbestimmten Fläche werden Maßnahmen abgeleitet, welche zum Schaffen einer Lebensgrundlage für die Soll-Insektenpopulation auf der vorbestimmten Fläche geeignet sind, wobei die Maßnahmen das Einbringen von Biostrukturen in die Fläche sind, und die in die Fläche eingebrachten Biostrukturen aus einer Gruppe ausgewählt sind, die Anhügelungen, Steinhaufen, Asthaufen, Trockenmauern, Steinkörbe, Kleingewässer, Bepflanzung wie insbesondere Bäume und Sträucher, Fassadenbegrünungen, Insektenhotels und Bachoffenlegung aufweist.

So kann die Vorrichtung zur Anwendung des Verfahrens zur Unterstützung der Erhaltung der Insektenpopulation über die Eingabe und/oder das Erfassen geeigneter Parameter die Anwendung eines Insektenvernichtungsmittels ausgehend von dessen Wirkung auf die Insektenpopulation die erforderliche Kompensation des Insektenverlusts ermitteln. Ausgehend von der von der Vorrichtung erfassten Ist-Insektenpopulation auf einer vorbestimmten Fläche ermittelt diese die erforderliche biologische Anpassung der vorbestimmten Fläche um eine Lebensgrundlage für die Soll-Insektenpopulation zu schaffen.

Weitere Vorteile, Merkmale und Anwendungsmöglichkeiten der vorliegenden Erfindung ergeben sich aus der nachfolgenden Beschreibung in Zusammenhang mit den Figuren.

Es zeigt, teilweise schematisch:
- Fig. 1:: eine beispielhafte als Flachdach ausgebildete vorbestimmte Fläche vor und nach Durchführung des erfindungsgemäßen Verfahrens zur Unterstützung der Erhaltung der Insektenpopulation jeweils in einer perspektivischen Ansicht;
- Fig. 2:: eine beispielhafte als Flachdach ausgebildete vorbestimmte Fläche nach Durchführung eines erfindungsgemäßen Verfahrens zur Unterstützung der Erhaltung der Insektenpopulation in der Draufsicht;
- Fig. 3:: eine beispielhafte erfindungsgemäße Vorrichtung zum Einsatz bei der Ausführung eines Verfahrens zur Unterstützung der Erhaltung der Insektenpopulation in einer schematischen Ansicht; und
- Fig. 4:: eine beispielhafte als Kraftfahrzeug ausgebildete Produkteinheit, auf die ein erfindungsgemäßes Verfahren angewendet werden kann in einer Seitenansicht (Fig. 4a) und einer Frontansicht (Fig. 4b).

Fig. 1 zeigt eine beispielhafte als Flachdach 1', 1 ausgebildete vorbestimmte Fläche 2, 3 vor und nach Durchführung eines erfindungsgemäßen Verfahrens zur Unterstützung der Erhaltung der Insektenpopulation jeweils in einer perspektivischen Ansicht.

Das Flachdach 1' wird im Rahmen der Durchführung eines erfindungsgemäßen Verfahrens als vorbestimmte Fläche 2 zur Unterstützung des Erhalts einer Insektenpopulation bestimmt.

Wie vielen Insekten das Flachdach 1' in dem Volumen im Wesentlichen über seiner Fläche 2 Lebensraum bereitstellen kann, und damit die Wertigkeit der Fläche 2 als Lebensraum für Insekten, hängt von der Ausprägung verschiedener Parameter ab, wie Bodenbeschaffenheit, Bewuchs, Vorhandensein von Biostrukturen, Feuchtigkeit, Sauerstoffgehalt und/oder Schadstoffbelastung der Luft, Temperatur, Sonneneinstrahlung und/oder Windgeschwindigkeit.

Im vorliegenden Beispiel ist die Fläche 2 des Flachdachs 1' vor der Durchführung eines erfindungsgemäßen Verfahrens mittels Teerfolie bzw. Bitumen versiegelt und weist keine anderen Strukturen, beispielsweise Biostrukturen, auf. Daher weist die Fläche 2 als Lebensraum für Insekten eine sehr geringe Wertigkeit auf, die in diesem Beispiel annäherungsweise mit Null gleichgesetzt wird.

Im hier beschriebenen Beispiel wird die letale Wirkung von 50.000 Produkteinheiten eines mechanischen Insektenbekämpfungsproduktes zu 100% kompensiert.

Diese Kompensation geschieht, indem gemäß einem Schritt des erfindungsgemäßen Verfahrens auf dem Flachdach 1' eine bessere Lebensgrundlage für eine Soll-Insektenpopulation geschaffen wird durch Anpassen einer entsprechenden Wertigkeit der vorbestimmten Fläche 2.

Durch das Schaffen dieser Lebensgrundlage wird die Fläche 2 in eine veränderte Fläche 3 des dann begrünten Flachdachs 1 transformiert, welche eine höhere Wertigkeit als Lebensraum für Insekten aufweist, als die Fläche 2 und damit eine höhere Anzahl bzw. Dichte an Insekten beherbergen kann.

Der in Figur 1 dargestellte vertikale Pfeil symbolisiert einen Zeitversatz zwischen einem Zeitpunkt vor der Anwendung des vorgeschlagenen Verfahrens und einem Zeitpunkt nach der Anwendung des vorgeschlagenen Verfahrens.

Wenn, wie im vorliegenden Fall, die Größe der Fläche 2 bzw. 3 vorbestimmt ist (hier 125m²), wird auf Basis der bestimmten Werte der letalen Wirkung der betrachteten Produkteinheiten und der auf der Fläche 2 Lebensraum findenden Insekten (hier näherungsweise Null) bestimmt, welche durchschnittliche Wertigkeit die Fläche 3 eines begrünten Flachdaches 1 aufweisen muss bzw. um wie viel die Wertigkeit in der Transformation von Fläche 2 in Fläche 3 verbessert werden muss, um eine Lebensgrundlage für die gewünschte Soll-Insektenpopulationen aufweisen zu können.

In einer weiteren Ausführungsform des Anwendungsbeispiels ist hingegen die zu erreichende Wertigkeit der vorbestimmten Fläche 3 festgelegt, auf Basis derer dann die notwendige Größe der vorbestimmten Fläche 3 ermittelt wird.

Zur Bestimmung der erforderlichen Wertigkeit der im Ausführungsbeispiel vorbestimmten Fläche werden Korrekturfaktoren einbezogen, welche den Umsatzzeitpunkt der Flächenaufwertung in Bezug auf den Insektenverlust und die Wirkungsentfaltung der Verbesserung der Fläche berücksichtigen. Ebenso wird bei der Bestimmung der Wertigkeit der Fläche auch ein Korrekturfaktor angesetzt, der zusätzliche Strukturen auf der Fläche berücksichtigt.

Vorliegend wird die Wertigkeit der Fläche 3 gegenüber der Wertigkeit der Fläche 2 zuerst dadurch verbessert, dass auf die versiegelte Oberfläche der Fläche 2 eine 10-12 cm dicke Schicht an mineralischem Dachgartensubstrat bzw. natürlichem Erdboden aufgebracht wird, welches/r unter anderem als Basis für die Einbringung weiterer, die Wertigkeit der Fläche 3 verbessernder, Biostrukturen dient.

Die Fläche 3 wird, abhängig von beeinflussenden Umgebungsparametern wie Sonnen- und Windexposition und/oder Möglichkeit der Bewässerung, gezielt mit weiteren Biostrukturen versehen, bis deren Kombination, Komposition, Positionierung und Menge in Verbindung mit der eingebrachten Bodenbeschaffenheit diejenige Wertigkeit des Lebensraums der Fläche 3 ermöglicht, die dem gewünschten Grad der Unterstützung des Erhalts der Insektenpopulation entspricht.

Als Biostrukturen werden in diesem Anwendungsbeispiel unter anderem pflanzliche Strukturen wie Büsche 4, Sträucher 11 und/oder blühende Pflanzen 10 verwendet, die den Insekten als Nahrungsquelle und/oder Nistplatz dienen können. Zudem können auf dem Dachgartensubstrat bzw. dem natürlichen Erdboden verschiedene Gräser 8 und andere Grünpflanzen eingebracht werden.

Auch totes pflanzliches Material wie Baumstümpfe und/oder -wurzeln 7 und/oder Rundholzabschnitte 6, kann den Lebensraum für verschiedene Insekten hinsichtlich Nahrung, Rückzugsmöglichkeiten und/oder Nistplätzen verbessern und wird vorliegend verwendet. Größere Mineralstrukturen 5, insbesondere poröse Steine, bilden ebenso Rückzugsmöglichkeiten für Insekten wie Aufschüttungen kleinerer Kieselsteine 9.

Durch die Verwendung von Biostrukturen 4-11 auf der vorbestimmten Fläche 3 kann auch der relative Anteil ökologisch wertvoller Insektenarten erhöht werden, was wiederum eine Erhöhung der Artenvielfalt und damit der Wertigkeit der vorbestimmten Fläche 3 zur Folge haben kann.

Fig. 2 zeigt eine beispielhafte als Flachdach 1 ausgebildete vorbestimmte Fläche 3 nach Durchführung eines erfindungsgemäßen Verfahrens zur Unterstützung der Erhaltung der Insektenpopulation in der Draufsicht.

In diesem Ausführungsbeispiel sind wiederum verschiedene Biostrukturen auf einen Untergrund der Fläche 3 aufgebracht, dessen Bodenbeschaffenheit von mineralischem Dachgartensubstrat bzw. natürlichem Erdboden geprägt ist.

Die Verwendung von Biostrukturen in diesem Ausführungsbeispiel unterscheidet sich von dem in Figur 1 beschriebenen Ausführungsbeispiel in der Kombination, Komposition, Positionierung und/oder Menge der einzelnen Biostrukturen.

Separate Bepflanzungsgruppen 12a, 12b, 12c umfassen, teilweise durch Rundholzabschnitte 6 eingefasst, Ansammlungen von Büschen 4, Sträuchern 11 und/oder blühenden Pflanzen 10. Auch eine Einbringung von Bäumen als Bepflanzung ausreichend tragfester Flachdächer 1 ist in diesem Ausführungsbeispiel vorgesehen.

Fig. 3 zeigt eine beispielhafte erfindungsgemäße Vorrichtung 20 zum Einsatz bei der Ausführung eines Verfahrens zur Unterstützung der Erhaltung der Insektenpopulation in einer Schnittansicht. Die Vorrichtung 20 weist unter anderem eine Wirkungserfassungseinrichtung 21, eine Mengenerfassungseinrichtung 23, eine Bestimmungseinrichtung, eine Ist-Erfassungseinrichtung 24 und eine Recheneinrichtung 26 auf.

Die Recheneinrichtung 26 ist bezüglich elektrischer Energie und bezüglich ein- und/oder beidseitiger Signalübertragung mit allen bereits genannten Erfassungseinrichtungen 21, 23, 24, 26 sowie mit wenigstens einem Eingabegerät 27, wie Tastatur bzw. Touchscreen, verbunden.

Die Wirkungserfassungseinrichtung 21 erfasst die letale Wirkung einer Produkteinheit auf die Insektenpopulation, und leitet aus wenigstens einem erfassten Wert die letale Wirkung einer Produkteinheit ab. Diese Erfassung kann beispielweise mittels eines Eingabegerätes 27 durch einen Experten erfolgen, oder aber auch über eine, beispielsweise sensorbasierte, Messvorrichtung 22. Die Messvorrichtung 22 erfasst Messwerte bzgl. dem Vorhandensein und/oder der Konzentration bestimmter Wirkstoffe von Produkteinheiten. Daraus wird in Verbindung mit in der Wirkungserfassungseinrichtung hinterlegten Daten und weiterer Angaben betreffend die Anwendung bzw. den Einsatz des Produkts die letale Wirkung einer Produkteinheit abgeleitet.

Die Mengenerfassungseinrichtung 23 erfasst die Menge der betrachteten Produkteinheiten. Diese Erfassung kann beispielweise mittels eines Eingabegerätes 27 durch einen Experten erfolgen.

Die nicht separat dargestellte Bestimmungseinrichtung ist in diesem Anwendungsbeispiel Teil der Recheneinrichtung 26 und bestimmt die Insektenpopulationsdifferenz infolge der Anwendung der Menge der Produkteinheiten unter Verwendung des ermittelten Wertes für die letale Wirkung einer Produkteinheit und hinterlegter Daten beispielsweise zu Gewicht und/oder Fortpflanzungscharakteristik bestimmter Insektenarten.

Die Ist-Erfassungseinrichtung 24 erfasst die Ist-Insektenpopulation auf einer vorbestimmten Fläche. Diese Erfassung kann beispielweise mittels eines Eingabegerätes 27 durch einen Experten erfolgen. Die Erfassung kann aber auch über die Sensoreinrichtung 25 erfolgen, welche die Insekten unmittelbar selbst erfasst oder mittels der Erfassung der Ist-Insektenpopulation einer bestimmten Insektenart oder der Ist-Insektenpopulation einer weiteren Insektenart oder der gesamten Ist-Insektenpopulation der Fläche 2 unmittelbar oder mittelbar erfassen.

Die Sensoreinrichtung 25 dieses Anwendungsbeispiel ist beispielsweise vorgesehen, wenigstens einen Sensor wie einen Feuchtigkeitssensor, Gas- und Feststoffsensor, Temperatursensoren, Helligkeitssensoren, Bewegungssensor zum Erfassen einer Windgeschwindigkeit, Farbsensor und/oder eine Kamera mit Bilderkennung aufzuweisen.

In die Recheneinrichtung 26 sind Werte aus den weiteren Einrichtungen 21, 23, 24, 26, 27 der Vorrichtung einlesbar. Aus den Werten für die Insektenpopulationsdifferenz und für die Ist-Insektenpopulation auf der vorbestimmten Fläche 2 die Soll-Insektenpopulation auf der vorbestimmten Fläche 3 bestimmt.

Im hier beschriebenen Anwendungsbeispiel sind in der Recheneinrichtung 26 Maßnahmen zur Anpassung der biologischen Bodenbeschaffenheit hinterlegt. Ausgehend von der erfassten Ist-Insektenpopulation auf der vorbestimmten Fläche 2 werden insbesondere aus den hinterlegten Maßnahmen geeignete Maßnahmen zum Schaffen einer Lebensgrundlage für die Soll-Insektenpopulation auf der vorbestimmten Fläche 3 abgeleitet.

Fig. 4 zeigt eine beispielhafte als Kraftfahrzeug 30 ausgebildete Produkteinheit, auf die ein erfindungsgemäßes Verfahren angewendet werden kann in einer Seitenansicht (Fig. 4a) und einer Frontansicht (Fig. 4b).

Das Kraftfahrzeug 30 bewegt sich mit einer Fahrgeschwindigkeit von 100 km/h in Richtung des in Fig. 4a eingezeichneten gestrichelten Pfeils.

Dabei wird eine Anströmfläche 35, welche sich im vorliegenden Ausführungsbeispiel im Wesentlichen aus einem Produkt aus der Breite Y und der Höhe Z errechnet, von dem Fahrtwind angeströmt, welcher aus der Fahrgeschwindigkeit des Kraftfahrzeugs 30 resultiert.

Wenn nun in dem Gebiet, durch das sich das Kraftfahrzeug 30 und damit die Anströmfläche 35 bewegt, ein Teil der Insektenpopulation aus Insekten und/ oder anderen Arthropoden fliegt, werden diese getötet, sobald sie insbesondere mit der Windschutzscheibe 31 oder dem Kühler 32 des Kraftfahrzeugs 30 kollidieren. Damit weist das Kraftfahrzeug 30 eine letale Wirkung auf eine Insektenpopulation auf.

Die letale Wirkung des Kraftfahrzeugs 30 auf die Insektenpopulation bei einer Jahreskilometerleistung von 16.000 km beträgt 28 g Insekten in einem Jahr. Die Insektenpopulationsdifferenz aufgrund der Wirkung dieses Kraftfahrzeugs 30 beträgt folglich 28 g Insekten.

Das in der Figur 1 gezeigte Flachdach 1' weist eine Fläche 2 auf, welche mittels Teerfolie bzw. Bitumen versiegelt ist und keine Biostrukturen aufweist, welche als Lebensgrundlage von Insekten dienen. Die Ist-Insektenpopulation auf der Fläche 2 wird daher mit näherungsweise Null bestimmt. Ziel ist es, dass die Fläche 3 des Flachdachs 1 ebenfalls für ein Jahr als Lebensgrundlage für die Insektenpopulationsdifferenz dient, welche aufgrund der Wirkung des Kraftfahrzeugs 30 zu Schaden kommt. Bei dem bei diesem Ausführungsbeispiel verwendeten einfachen Rechenverfahren entspricht die Soll-Insektenpopulation auf der Fläche 3 der Insektenpopulationsdifferenz, da die Ist-Insektenpopulation auf der Fläche 2 näherungsweise Null ist.

In einem weiteren Schritt des Ausführungsbeispiels für die Anwendung des vorgeschlagenen Verfahrens wird durch Anpassen der biologischen Beschaffenheit der Fläche 2 durch das Schaffen einer geeigneten Bodenstruktur und dem Einbringen von Bepflanzungen wie Büschen 4 und Sträuchern 11 eine Fläche 3 geschaffen, welche einer Soll-Insektenpopulation von 28 g Insekten in einem Jahr eine Lebensgrundlage bildet. Das Kraftfahrzeug 30, für dessen letale Wirkung auf die Insektenpopulation eine Erhaltungsmaßnahme durchgeführt wurde, wird im Anschluss an die Durchführung der Maßnahme mit einer entsprechenden Markierung versehen.

## Patentansprüche

1. Verfahren zur Unterstützung der Erhaltung der Insektenpopulation mit folgenden Schritten:
- Ermitteln der letalen Wirkung einer Produkteinheit (30) auf eine erste Insektenpopulation,
- Bestimmen einer Insektenpopulationsdifferenz aufgrund der Wirkung einer bestimmten Menge von Produkteinheiten (30) auf diese erste Insektenpopulation,
- Ermitteln einer zweiten Insektenpopulation, der Ist-Insektenpopulation auf einer vorbestimmten Fläche (2, 3),
- Bestimmen einer Soll-Insektenpopulation auf der vorbestimmten Fläche (2, 3), welche die Summe ist aus der Ist-Insektenpopulation auf der vorbestimmten Fläche (2, 3) und der wahlweise um einen Faktor vergrößerten oder verringerten Insektenpopulationsdifferenz, und
- Schaffen einer Lebensgrundlage für die Soll-Insektenpopulation auf der vorbestimmten Fläche (2, 3) durch Anpassen der biologischen Beschaffenheit der vorbestimmten Fläche (2, 3), **dadurch gekennzeichnet, dass** die Wertigkeit, also die biologische Beschaffenheit der vorbestimmte Fläche (2, 3) mithilfe einer Einbringung von Biostrukturen (4, 5, 6, 7, 8, 9, 10, 11, 12) in die Fläche (2, 3) erhöht wird, wobei die in die Fläche (2, 3) eingebrachten Biostrukturen aus einer Gruppe ausgewählt sind, die Anhügelungen, Steinhaufen (5, 9), Asthaufen (7), Trockenmauern (6), Steinkörbe, Kleingewässer, Bepflanzung (4, 8, 10, 11) wie insbesondere Bäume und Sträucher (4), Fassadenbegrünungen, Insektenhotels und Bachoffenlegung aufweist.

2. Verfahren zur Unterstützung der Erhaltung der Insektenpopulation nach Anspruch 1, **dadurch gekennzeichnet, dass** die Wirkung einer Produkteinheit (30) mechanisch und/ oder chemisch ist.

3. Verfahren zur Unterstützung der Erhaltung der Insektenpopulation nach einem der vorhergehenden Ansprüche, **dadurch gekennzeichnet, dass** die Ist-Insektenpopulation auf einer vorbestimmten Fläche (2, 3) aufgrund der Wertigkeit, also der biologischen Beschaffenheit der Fläche (2, 3) bestimmt wird.

4. Verfahren zur Unterstützung der Erhaltung der Insektenpopulation nach einem der vorhergehenden Ansprüche, **dadurch gekennzeichnet, dass** die Ist-Insektenpopulation auf einer vorbestimmten Fläche (2, 3) mithilfe wenigstens eines Sensors (25) erfasst wird, wobei der Sensor (25) die Insekten vorzugsweise selbst erfasst.

5. Verfahren zur Unterstützung der Erhaltung der Insektenpopulation nach Anspruch 4, **dadurch gekennzeichnet, dass** wenigstens ein Sensor (25) wenigstens einen Parameter erfasst, aufgrund welchem die Wertigkeit, also die biologische Beschaffenheit der vorbestimmten Fläche (2, 3) ermittelt und die Ist-Insektenpopulation auf dieser Fläche (2, 3) bestimmbar ist.

6. Verfahren zur Unterstützung der Erhaltung der Insektenpopulation nach Anspruch 5, **dadurch gekennzeichnet, dass** der wenigstens eine von dem mindestens einem Sensor (25) erfasste Parameter aus einer Gruppe ausgewählt ist, welche den Bewuchs, die Feuchtigkeit, den Sauerstoffgehalt und/ oder die Schadstoffbelastung der Luft, die Temperatur, die Sonneneinstrahlung, die Windgeschwindigkeit, Farben, die Bodenbeschaffenheit und das Vorhandensein von Biostrukturen (4, 5, 6, 7, 8, 9, 10, 11, 12) aufweist.

7. Verfahren zur Unterstützung der Erhaltung der Insektenpopulation nach einem der vorhergehenden Ansprüche, **dadurch gekennzeichnet, dass** der Faktor ein Korrekturfaktor ist, um einen ökologischen Wert der Artenvielfalt zu berücksichtigen, wobei besonders wertvollen beziehungsweise nützlichen Insektenarten ein höherer Wert des Korrekturfaktors zugewiesen wird, als Insektenarten, die für die Artenvielfalt des Ökosystems weniger wertvoll eingeschätzt werden.

8. Verfahren zur Unterstützung der Erhaltung der Insektenpopulation nach Anspruch 1, **dadurch gekennzeichnet, dass** der Faktor den zeitlichen Versatz zwischen der Anwendung der Produkteinheit und der Anpassung der biologischen Beschaffenheit der vorbestimmten Fläche, und/ oder die Wirkungsentfaltung der Anpassung und/ oder die Wirkung der Strukturvielfalt der biologischen Beschaffenheit berücksichtigt.

9. Verfahren zur Unterstützung der Erhaltung der Insektenpopulation nach einem der vorhergehenden Ansprüche, **dadurch gekennzeichnet, dass** die erforderliche Wertigkeit, also die erforderliche biologische Beschaffenheit der vorbestimmten Fläche (2, 3) in Abhängigkeit der Soll-Insektenpopulation ermittelt wird.

10. Verfahren zur Unterstützung der Erhaltung der Insektenpopulation nach einem der vorhergehenden Ansprüche, **dadurch gekennzeichnet, dass** die Wertigkeit, also die biologische Beschaffenheit der vorbestimmten Fläche (2, 3) mithilfe einer Veränderung der Bodenbeschaffenheit erhöht wird.

11. Verfahren zur Unterstützung der Erhaltung der Insektenpopulation nach einem der vorhergehenden Ansprüche, **dadurch gekennzeichnet, dass** die Produkteinheit (30) mit einer Markierung versehen wird, die anzeigt, dass das Verfahren für dessen letale Wirkung durchgeführt wurde.

12. Vorrichtung (20) zur Anwendung eines Verfahrens zur Unterstützung der Erhaltung der Insektenpopulation, gemäß einem der vorhergehenden Ansprüche, aufweisend:
- eine Wirkungserfassungseinrichtung (21) zum Erfassen der letalen Wirkung einer Produkteinheit (30) auf eine erste Insektenpopulation, welche vorzugsweise aus wenigstens einem erfassten Wert die letale Wirkung einer Produkteinheit (30) ableitet,
- eine Mengenerfassungseinrichtung (23) zum Erfassen der Menge der betrachteten Produkteinheiten (30),
- eine Bestimmungseinrichtung zum Bestimmen der Insektenpopulationsdifferenz infolge der Anwendung der Menge der Produkteinheiten (30),
- wenigstens eine Ist-Erfassungseinrichtung (24) zum Erfassen einer zweiten Insektenpopulation, der Ist-Insektenpopulation auf einer vorbestimmten Fläche (2, 3) und
- eine Recheneinrichtung (26), in welche Werte aus den weiteren Einrichtungen (21, 23, 24, 27) der Vorrichtung (20) einlesbar sind und welche wenigstens aus den Werten für die Insektenpopulationsdifferenz und für die Ist-Insektenpopulation auf der vorbestimmten Fläche (2, 3) die Soll-Insektenpopulation auf der vorbestimmten Fläche (2, 3) bestimmt,
wobei in der Recheneinrichtung (26) Maßnahmen zur Anpassung der biologischen Bodenbeschaffenheit hinterlegt sind und ausgehend von der erfassten Ist-Insektenpopulation auf der vorbestimmten Fläche (2, 3) abgeleitet werden, zum Schaffen einer Lebensgrundlage für die Soll-Insektenpopulation auf der vorbestimmten Fläche (2, 3), wobei die Maßnahmen das Einbringen von Biostrukturen in die Fläche sind, und die in die Fläche (2, 3) eingebrachten Biostrukturen aus einer Gruppe ausgewählt sind, die Anhügelungen, Steinhaufen (5, 9), Asthaufen (7), Trockenmauern (6), Steinkörbe, Kleingewässer, Bepflanzung (4, 8, 10, 11) wie insbesondere Bäume und Sträucher (4), Fassadenbegrünungen, Insektenhotels und Bachoffenlegung aufweist.

## Claims

1. A method for supporting the preservation of the insect population which comprises the following steps:
- ascertaining the lethal effect of a product unit (30) on a first insect population,
- determining a difference in insect population as a result of the effect of a certain quantity of product units (30) on said first insect population,
- ascertaining a second insect population, the actual insect population on a predetermined area (2, 3),
- determining a target insect population on the predetermined area (2, 3) which is the sum of the actual insect population on the predetermined area (2, 3) and the difference in insect population either increased or decreased by a factor, and
- creating a basis of life for the target insect population on the predetermined area (2, 3) by adapting the biological nature of the predetermined area (2, 3), **characterized in that** the quality value, i.e. the biological nature of the predetermined area (2, 3), is increased by means of introducing biostructures (4, 5, 6, 7, 8, 9, 10, 11, 12) into the area (2, 3), wherein the biostructures introduced into the area (2, 3) are selected from among a group comprising mounds, cairns (5, 9), clumps of branches (7), dry stone walls (6), gabions, small bodies of water, plantings (4, 8, 10, 11) such as in particular trees and shrubs (4), façade vegetation, insect hotels and stream openings.

2. The method for supporting the preservation of the insect population according to claim 1, **characterized in that** the effect of a product unit (30) is mechanical and/or chemical.

3. The method for supporting the preservation of the insect population according to one of the preceding claims, **characterized in that** the actual insect population on a predetermined area (2, 3) is determined on the basis of the quality value, i.e. the biological nature of the area (2, 3)..

4. The method for supporting the preservation of the insect population according to one of the preceding claims, **characterized in that** the actual insect population on a predetermined area (2, 3) is detected by means of at least one sensor (25), wherein the sensor (25) preferably detects the insects themselves.

5. The method for supporting the preservation of the insect population according to claim 4, **characterized in that** at least one sensor (25) detects at least one parameter on the basis of which the quality value, i.e. the biological nature of the predetermined area (2, 3) is ascertained and the actual insect population on said area (2, 3) can be determined.

6. The method for supporting the preservation of the insect population according to claim 5, **characterized in that** the at least one parameter detected by the at least one sensor (25) is selected from among a group comprising the vegetation, the humidity, the oxygen content and/or the pollution of the air, the temperature, the solar radiation, the wind speed, colors, the soil properties and the presence of biostructures (4, 5, 6, 7, 8, 9, 10, 11, 12).

7. The method for supporting the preservation of the insect population according to one of the preceding claims, **characterized in that** the factor is a correction factor to allow for an environmental value of the biodiversity, wherein particularly valuable or useful species of insects are assigned a higher correction factor value than species of insects judged as being less valuable to the biodiversity of the ecosystem.

8. The method for supporting the preservation of the insect population according to claim 1, **characterized in that** the factor takes into account the time lag between the application of the product unit and the adaptation of the biological nature of the predetermined area and/or the development of the adaptation effect and/or the effect of the structural diversity of the biological nature.

9. The method for supporting the preservation of the insect population according to one of the preceding claims, **characterized in that** the required quality value, i.e. the required biological nature of the predetermined area (2, 3) is ascertained as a function of the target insect population.

10. The method for supporting the preservation of the insect population according to one of the preceding claims, **characterized in that** the quality value, i.e. the biological nature of the predetermined area (2, 3) is increased by changing the properties of the soil.

11. The method for supporting the preservation of the insect population according to one of the preceding claims, **characterized in that** the product unit (30) is provided with a marking indicating that the method was undertaken for its lethal effect.

12. An apparatus (20) for implementing a method for supporting the preservation of the insect population according to one of the preceding claims, comprising:
- an effect detection device (21) for detecting the lethal effect of a product unit (30) on a first population of insects which derives the lethal effect of a product unit (30) preferably from at least one detected value,
- a quantity detection device (23) for detecting the quantity of considered product units (30),
- a determination device for determining the difference in the insect population resulting from applying the quantity of product units (30),
- at least one actual-value detection device (24) for detecting a second insect population, the actual insect population on a predetermined area (2, 3), and
- a computing device (26) into which values from the other devices (21, 23, 24, 27) of the apparatus (20) can be read and which determines the target insect population on the predetermined area (2, 3) at least from the values for the difference in insect population and the actual insect population on the predetermined area (2, 3),
wherein measures for the adapting of the biological properties of the soil are stored in the computing device (26) and derived from the detected actual insect population on the predetermined area (2, 3) to create a basis of life for the target insect population on the predetermined area (2, 3), wherein the measures are the introducing of biostructures into the area, and the biostructures introduced into the area (2, 3) are selected from among a group comprising mounds, cairns (5, 9), clumps of branches (7), dry stone walls (6), gabions, small bodies of water, plantings (4, 8, 10, 11) such as in particular trees and shrubs (4), façade vegetation, insect hotels and stream exposures.

## Revendications

1. Procédé pour aider à la conservation de la population d'insectes comprenant les étapes suivantes :
- déterminer l'action létale d'une unité produit (30) sur une première population d'insectes,
- déterminer une différence au sein de la population d'insectes sur la base de l'action d'une certaine quantité d'unités produits (30),
- déterminer une deuxième population d'insectes sur une surface prédéfinie (2, 3),
- déterminer une population d'insectes théorique sur la surface prédéfinie (2, 3), laquelle est la somme de la population d'insectes réelle sur la surface prédéfinie (2, 3), et de la différence au sein de la population d'insectes au choix augmentée ou diminuée d'un facteur,
et
- créer les subsistances nécessaires à la population d'insectes théorique sur la surface prédéfinie (2, 3) par adaptation de la constitution biologique de la surface prédéfinie (2,3),
**caractérisé en ce que** la valence, autrement dit la constitution biologique de la surface prédéfinie (2, 3) est augmentée grâce à l'apport de biostructures (4, 5, 6, 7, 8, 9, 10, 11, 12) dans la surface (2, 3), les biostructures apportées dans la surface (2, 3) étant sélectionnées dans une groupe présentant des mises en tas, des tas de pierres (5, 9), des tas de branches (7), des murs de pierres sèches (6), des gabions, des petits plans d'eau, de la végétation (4, 8, 10, 11) telle que, en particulier, des arbres et des arbustes (4), des végétalisations de façade, des hôtels à insectes et un ruisseau remis à ciel ouvert.

2. Procédé pour aider à la conservation de la population d'insectes selon la revendication 1, **caractérisé en ce que** l'action d'une unité de produit (30) est mécanique et/ou chimique.

3. Procédé pour aider à la conservation de la population d'insectes selon l'une quelconque des revendications précédentes, **caractérisé en ce que** la population d'insectes réelle sur une surface prédéfinie (2, 3) est détectée sur la base de la valence, autrement dit de la constitution biologique de la surface (2, 3).

4. Procédé pour aider à la conservation de la population d'insectes selon l'une quelconque des revendications précédentes, **caractérisé en ce que** la population d'insectes réelle sur une surface prédéfinie (2, 3) est détectée à l'aide d'au moins un capteur (25), le capteur (25) détectant les insectes de préférence lui-même.

5. Procédé pour aider à la conservation de la population d'insectes selon la revendication 4, **caractérisé en ce qu'**au moins un capteur (25) détecte au moins un paramètre sur la base duquel la valence, autrement dit la constitution de la surface prédéfinie (2 3), est déterminée et la population d'insectes réelle sur cette surface (2, 3) peut être déterminée.

6. Procédé pour aider à la conservation de la population d'insectes selon la revendication 5, **caractérisé en ce que** l'au moins un paramètre détecté par l'au moins un capteur (25) est sélectionné dans un groupe présentant la couverture végétale, l'humidité, la teneur en oxygène et/ou la pollution de l'air, la température, le rayonnement solaire, la vitesse du vent, les couleurs, la constitution de sol et la présence de biostructures (4, 5, 6, 7, 8, 9, 10, 11, 12).

7. Procédé pour aider à la conservation de la population d'insectes selon l'une quelconque des revendications précédentes, **caractérisé en ce que** le facteur est un facteur de correction pour tenir compte de la valeur écologique de la diversité des espèces, une plus grande valeur du facteur de correction étant attribuée en particulier à des espèces d'insectes particulièrement précieuses respectivement utiles en tant qu'espèces d'insectes estimées moins précieuses pour la diversité des espèces de l'écosystème.

8. Procédé pour aider à la conservation de la population d'insectes selon la revendication 1, **caractérisé en ce que** le facteur tient compte du décalage dans le temps entre l'application de l'unité de produit et l'adaptation de la constitution biologique de la surface prédéfinie et/ou le déploiement des actions de l'adaptation et/ou l'action de la diversité structurelle de la constitution biologique.

9. Procédé pour aider à la conservation de la population d'insectes selon l'une quelconque des revendications précédentes, **caractérisé en ce que** la valence nécessaire, autrement dit la constitution biologique nécessaire de la surface prédéfinie (2, 3) est déterminée en fonction de la population d'insectes théorique.

10. Procédé pour aider à la conservation de la population d'insectes selon l'une quelconque des revendications précédentes, **caractérisé en ce que** la valence, autrement dit la constitution biologique nécessaire de la surface prédéfinie (2, 3) est augmentée à l'aide d'une modification de la constitution du sol.

11. Procédé pour aider à la conservation de la population d'insectes selon l'une quelconque des revendications précédentes, **caractérisé en ce que** l'unité de produit (30) est dotée d'une marque qui indique que le procédé a été réalisé pour l'action l'étale de celui-ci.

12. Dispositif (20) pour l'application d'un procédé d'aide à la conservation de la population d'insectes selon l'une quelconque des revendications précédentes présentant :
- un équipement de détection d'actions (21) pour détecter l'action létale d'une unité de produit (30) sur une première population d'insectes qui déduit l'action létale d'une unité de produit (30) de préférence d'au moins une valeur détectée,
- un équipement de détection de quantités (23) pour détecter la quantité des unités de produits considérées (30),
- un équipement de détermination pour déterminer la différence au sein de la population d'insectes suite à l'application de la quantité d'unités de produits (30),
- au moins un équipement de détection réelle (24) pour la détection d'une deuxième population d'insectes, la population d'insectes réelle sur une surfaces prédéfinie (2, 3) et
- un équipement de calcul (26) dans lequel les valeurs provenant des autres équipements (21, 23, 24, 27) du dispositif (20) peuvent être lues et qui calcule la population d'insectes théorique sur la surface prédéfinie (2, 3) au moins à partir des valeurs de la différence au sein de la population d'insectes et de la population d'insectes réelle sur la surface prédéfinie (2, 3), des mesures d'adaptation de la constitution biologique du sol étant enregistrées dans l'équipement de calcul (26) et dérivées de la population d'insectes réelle sur la surface prédéfinie (2, 3), pour créer les substances pour la population d'insectes théorique sur la surface prédéfinie (2, 3), les mesures destinées à l'apport de biostructures dans la structure, et les biostructures apportées dans la surface (2, 3) étant sélectionnées dans un groupe présentant des mises en tas, des tas de pierres (5, 9), des tas de branches (7), des murs de pierres sèches (6), des gabions, des petits plans d'eau, de la végétation (4, 8, 10, 11) telle que, en particulier, des arbres et des arbustes (4), des végétalisations de façade, des hôtels à insectes et un ruisseau remis à ciel ouvert.
